# FASCICULE DE BREVET EUROPEEN

(11) **EP 1 156 013 B1**
(45) Date de publication et mention de la délivrance du brevet: **15.08.2007**
(21) Numéro de dépôt: 01401244.7
(22) Date de dépôt: 15.05.2001
(51) Int. Cl.: C02F 1/02, C02F 1/76

(54) **Procédé continu de désinfection contre la légionellose dans les équipements à eau chaude sanitaire**
Kontinuierliches Desinfektionsverfahren gegen Legionellosis in Warmwasserversorgungseinrichtungen
Continuous process for disinfection of legionellosis in hot water supply systems

(30) Priorité: 16.05.2000 FR 0006599
(43) Date de publication de la demande: 21.11.2001
(73) Titulaire: Institut Français du Pétrole, 92852 Rueil-Malmaison Cedex (FR); Lacaze S.A., 46120 Leyme (FR)
(72) Inventeur: Lacaze, Pierre, 46120 Leyme (FR); Hendou, Mouloud, 46400 Bannes (FR); Collin, Jean-Claude, 92500 Rueil Malmaison (FR)

(56) Documents cités:
- EP-A- 0 594 020
- WO-A-99/07641
- FR-A- 2 644 155

## Description

La présente invention concerne le domaine de la désinfection et de la lutte contre la légionellose dans les équipements de stockage et de production et dans l'ensemble des circuits de distribution d'eau chaude sanitaire.
Dans la présente description, on désignera de façon générique par « légionellose » les affections médicales causées par la bactérie *Legionella pulmophila* et par d'autres bactéries du même type (voir notamment *La Recherche* n° 141, février 1983, p. 146).
On connaît des dispositifs de production de chlore libre à partir d'électrolytes constitués d'eau et de sels (par exemple le chlorure de sodium) à fortes concentrations en sels (par exemple de 65 à 150 g/l).
On connaît aussi des dispositifs d'électrolyse à compartiments séparés destinés à transformer électro-chimiquement une solution d'eau et de sels avec obtention de chlore moléculaire gazeux (Cl₂) dans les chambres anodiques.
Ces types d'installations sont destinés à la production de solutions contenant du chlore libre ou à la production de chlore gazeux en vue de leur stockage et d'une opération de désinfection en mode discontinu. Par ailleurs, afin d'obtenir de bons rendements de transformation, la solution électrolytique est recyclée dans le réacteur électrolytique, ce qui empêche l'utilisation de ces techniques pour l'injection en continu du chlore libre dans l'eau.
Les solutions électrolytiques utilisées dans ces dispositifs (fortement chargées en sel) engendrent, après transformation électrochimique, des produits contenant de 30 à 70 g/l de chlorure. L'utilisation de ces produits pour la désinfection et la lutte contre la légionellose dans les équipements de production et de stockage et dans les circuits de distribution d'eau chaude sanitaire pose des problèmes de corrosion liés à l'augmentation du taux de chlorure dans l'eau suite à l'injection de ces produits.
Il faut signaler aussi que les solutions issues des techniques électrolytiques précitées sont caractérisées par un pH alcalin, défavorable à la formation du chlore libre actif et à l'effet bactéricide.
Dans le domaine de la lutte anti-légionellose, on connaît aussi le traitement discontinu par injection du chlore libre commercial.
On connaît le traitement anti-légionellose réalisé par adjonction de bioxyde de chlore produit dans des réacteurs par l'action de l'acide sur des chlorites.
L'expérience montre que ces traitements sont insatisfaisants, suite à l'acclimatation de la bactérie dans ces milieux.
Enfin, l'état de l'art relate aussi un traitement curatif occasionnel par choc thermique à 70 °C pendant une demi-heure, ce traitement n'assurant en aucun cas l'absence permanente de la bactérie dans l'eau.

Le document EP0594020 A1 décrit une installation de chauffage d'eau à usage sanitaire et d'élimination des légionelles en circuit fermé à sens unique comprenant une conduite de distribution, une conduite de circulation et un circuit de désinfection de l'eau à une température de l'ordre de 65-70°C.

Le procédé de la présente invention présente en revanche l'avantage de permettre d'assurer en permanence la présence du chlore libre dans l'eau chaude distribuée, ce qui induit un effet préventif permanent notamment dans les conduites de distribution.

La présente invention a donc pour objet un procédé continu de désinfection et de lutte contre la légionellose dans les équipements de stockage et de production et les circuits de distribution d'eau chaude sanitaire qui comprend :
- une section (B) de choc thermique permanent assurant la fonction curative et préventive anti-légionellose, mettant en oeuvre un réservoir d'admission de l'eau et de préchauffage, un réservoir de choc thermique à une température allant de 85°C jusqu'à 109 °C et un moyen d'échange de chaleur, de façon telle que définie dans la revendication 1
- éventuellement, l'injection permanente dans l'eau introduite dans la section (B) d'un débit d'une solution électrolytique obtenue par une électro-chloration continue menée dans une section (A) de production de chlore libre par électrolyse (permettant d'assurer la présence de chlore libre dans l'eau chaude à une concentration d'environ 1 à 2 mg/l) ;
- le procédé pouvant également comprendre une section (C) d'électrolyse permanente assurant la protection cathodique contre la corrosion des réservoirs utilisés dans la section (B), par courant imposé, et la génération continue du chlore libre au sein des réservoirs, pour compenser l'effet de dismutation du chlore libre favorisé par les températures élevées.

La présente invention sera décrite plus en détail ci-après, en relation avec les figures 1 à 4 annexées, parmi les quelles :
- la figure 1 montre le schéma général de la section (B) du procédé selon l'invention, ainsi que de la section (A) ;
- la figure 2A illustre schématiquement la cathode et la figure 2B l'anode du réacteur d'électrolyse du dispositif de production du chlore libre utilisé dans la section (A) ;
- la figure 3 illustre les équilibres HClO et ClO⁻ (pourcentages massiques de distribution en fonction du pH) ; et
- la figure 4 montre un exemple de régulation de l'intensité de courant (I) dans le réacteur d'électrolyse de la section (A) en fonction du débit d'eau à traiter (D).

Dans la section d'opération (B) qui constitue l'objet principal de l'invention, l'eau qui peut contenir du chlore libre, par exemple après injection d'une solution électrolytique telle que produite par électrolyse dans la section (A) telle que décrite plus loin, subit en continu un choc thermique à un niveau de température pouvant aller de 85°C jusqu'à 109 °C, ce qui permet d'assurer une désinfection et une stérilisation continue et totale de l'eau.

Selon cet aspect de l'invention, on met en oeuvre un dispositif qui comprend un réservoir d'admission de l'eau, le cas échéant rectifiée en chlore libre, un autre réservoir de choc thermique, un échangeur de chaleur, une vanne de mélange et de distribution, des moyens de mesure de la température de distribution, des moyens de réglage de la température de distribution d'eau chaude, comme défini dans la revendication 10.

Le circuit de traitement pour la mise en oeuvre de cette section (B) peut être schématisé à titre indicatif et nullement limitatif de la manière suivante :

L'eau du réseau, éventuellement après avoir subi une adjonction de chlore libre en permanence, est admise dans le réservoir d'admission, sous une pression pouvant aller part exemple jusqu'à 10 bar, ledit réservoir servant de tampon et de préchauffage par échange thermique avec l'eau contenue dans le réservoir de choc thermique au moyen d'un échangeur de chaleur. A la sortie de l'échangeur, l'eau est admise dans le réservoir doté d'un moyen de chauffage assurant un choc thermique permanent de l'eau à un niveau de température pouvant atteindre 109 °C. L'eau sortant du réservoir de choc thermique est ensuite refroidie par échange avec l'eau entrante au travers de l'échangeur, avant d'être distribuée à la température désirée.

Ce circuit de traitement permet d'assurer une dépense énergétique n'excédant pas celle nécessaire au chauffage de l'eau de la température ambiante à la température de distribution.

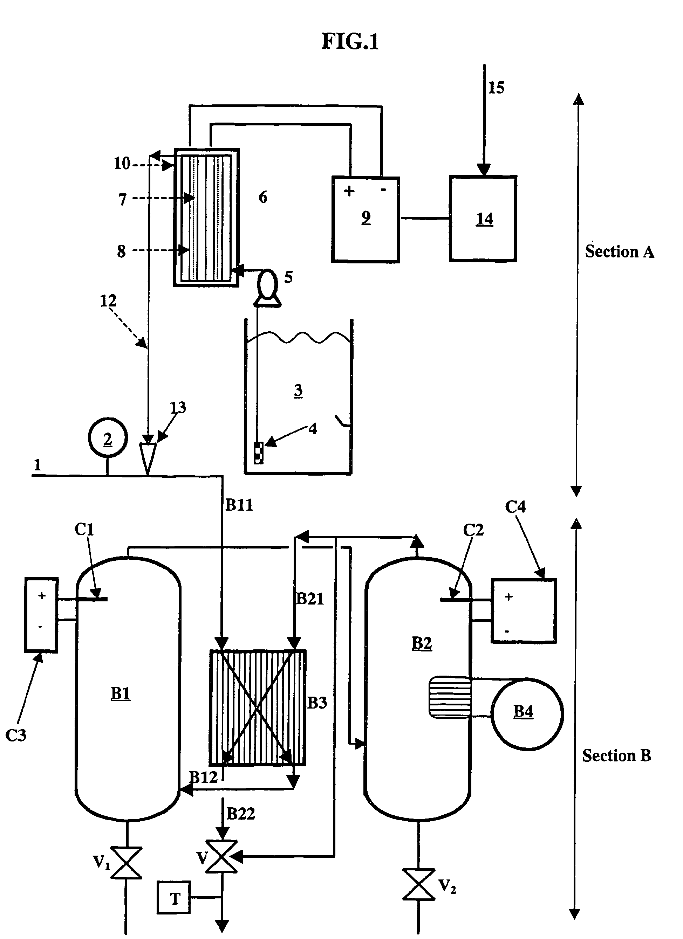

Si l'on se réfère à la figure 1 (section B), la conduite 1 alimente le réservoir B1 sous pression (allant par exemple jusqu'à 10 bar) en réalisant le circuit B11-B12, la circulation de l'eau se faisant par l'intermédiaire de l'échangeur B3. Ledit réservoir B1 permet de stocker l'eau préchauffée à une température par exemple de 15 à 65 °C, de préférence d'environ 35 °C. Le volume de B1 permet de mieux contrôler le procédé.

L'eau issue du réservoir B1 rejoint le deuxième réservoir B2 siège du choc thermique. Le réservoir B2 est doté d'un moyen de chauffage B4 dont la puissance est suffisante pour un chauffage et un maintien de sa température à une valeur pouvant aller de 85°C jusqu'à 109 °C.

L'eau du réseau ayant éventuellement subi une adjonction contrôlée de chlore libre au niveau de la sonde d'injection 13, subit un préchauffage dans l'échangeur B3 suivant le circuit hydraulique B11-B12 puis un choc thermique dans le réservoir B2 ; elle est ensuite véhiculée dans l'échangeur B3 à travers les conduites B21-B22 pour céder son excès calorifique dû au choc thermique en préchauffant une nouvelle quantité d'eau froide entrant dans le circuit de traitement.

L'eau sortant de l'échangeur B3 (c'est-à-dire du circuit B21-B22) passe à travers une vanne mélangeuse V de type « trois-voies », qui permet la régulation de la température de distribution de l'eau chaude traitée par admission d'une proportion d'eau très chaude du réservoir B2 contrôlée par le régulateur de température T. Ce cas de figure peut être rencontré dans le cas de traitement de faibles débits d'eau.

Selon l'invention, il est possible de mettre en jeu un ou plusieurs réservoirs B1 ainsi que un ou plusieurs réservoirs B2.

Conformément à l'invention, ledit réservoir B2 doit permettre un temps de séjour pour l'eau d'au moins une demi-heure. Il en ressort que le nombre de réservoirs B1 et B2, et leurs capacités volumique et thermique sont choisis en fonction des besoins : la consommation d'eau chaude et le niveau de température requis.

Dans le dispositif pour la mise en oeuvre de la section (B) du procédé de l'invention, les réservoirs B1 et B2 peuvent être des cuves en acier peint. Dans ce dispositif, l'échangeur de chaleur B3 peut être par exemple un échangeur à plaques ou encore un échangeur à tubes et calandre. La vanne de mélange peut être une vanne de mélange trois-voies.

La présence du réservoir B1 est fondamentale pour la bonne marche du procédé électro thermo choc. En effet ce réservoir est le siège d'un pré-détartrage de l'eau par effet thermique obtenu avec le préchauffage à travers l'échanger B3.

Ce détartrage est renforcé par la présence de la protection cathodique C1 par courant imposé. En effet, la circulation d'un courant continu engendre une augmentation de la cinétique des réactions chimiques d'entartrage, l'électrolyse changeant également l'aspect du tartre (tartre pulvérulent).

Le détartrage assure deux fonctions différentes :

Tout d'abord, l'eau distribuée dans le réseau d'eau chaude sanitaire est exempte de tartre, ce qui limite les interventions de maintenance.

Enfin une source de nutrition pour la légionelle est supprimée. En effet, il est notoire que la légionelle trouve ses éléments nutritifs dans les dépôts de tartre, créant un nid de croissance de la légionelle dans cet élément.

D'autre part, chaque réservoir B ou B2 est, de préférence, muni en partie inférieure d'une vanne de débourbage (V1 sur B1, V2 sur B2), éventuellement automatique, qui permet d'extraire de façon continue le tartre accumulé. Il est à noter que le procédé selon l'invention préserve toujours le sens d'écoulement des fluides, évitant au réseau eau chaude sanitaire d'être pollué par les dépôts de toute nature, notamment tartreux.

Dans l'eau du réseau à traiter par l'opération de choc thermique selon l'invention, un apport de chlore peut être effectué sous différentes formes, par exemple sous forme de chlore gazeux, sous forme liquide (par exemple Eau de Javel) ou solide (par exemple hypochlorite de potassium).

D'une manière préférée selon l'invention, l'apport de chlore dans l'eau à traiter est effectué par injection d'une solution électrolytique contenant du chlore libre, produite par exemple par mise en oeuvre d'une section d'électrolyse (A) telle que décrite dans la demande de brevet français déposée par les demanderesses sous le numéro FR 00/00 598, le 18 janvier 2000 ( = EP1118586/ FR280384). La section d'électrolyse (A) permet l'obtention par électrolyse d'une solution de désinfection spécifique aux équipements de stockage ou de production et aux circuits de distribution d'eau chaude. Comme il ressort de la description qui suit, certains avantages de la mise en oeuvre de la section (A) résident notamment dans les caractéristiques de la solution électrolytique et dans la possibilité d'automatisation complète du fonctionnement assurant une désinfection permanente sans risque des effets secondaires néfastes.

On procède plus particulièrement comme décrit ci-après.

On effectue l'électrolyse d'un électrolyte renfermant de l'eau et du sel (par exemple le chlorure de sodium) à une concentration ne dépassant pas 30 g/l, plus particulièrement inférieure à 20 g/l, ainsi que de l'acide chlorhydrique en quantité convenable pour atteindre un pH compris entre environ 4 et 5. La solution obtenue par l'électrolyse dudit électrolyte, fournit par électrolyse un produit contenant du chlore libre par exemple à une concentration de 3 g/l et présentant un pH sensiblement neutre, favorable à la formation du chlore libre actif et à l'effet bactéricide.

Le dispositif pour la mise en oeuvre de cette section (A) comprend un bac de préparation d'un électrolyte, une pompe d'injection de l'électrolyte dans un réacteur d'électrolyse contenant une anode et une cathode, une conduite d'injection de la solution électrolysée dans une canalisation d'eau, des moyens de mesure du débit d'eau en circulation dans ladite canalisation, des moyens de réglage de l'intensité de courant entre l'anode et la cathode et du débit d'injection de l'électrolyte.

La mise en oeuvre de la section (A) permet l'injection permanente du chlore libre dans l'eau destinée au chauffage sans modifier son pH et avec une augmentation du taux de chlorure ne dépassant pas 10 mg/l, ce qui préserve les équipements contre la corrosion.

Le bac de préparation utilisé dans le dispositif pour la mise en oeuvre de la section (A) peut comporter une sonde de mesure du pH de la solution électrolytique et comporter des moyens de réglage du pH de l'électrolyte. Les moyens de régulation du débit d'injection et/ou de l'intensité de courant peuvent agir en fonction du débit d'eau froide dans la canalisation.

La solution électrolytique peut comprendre de l'eau, du chlorure de sodium et de l'acide chlorhydrique. La concentration en chlorure de sodium peut ne pas excéder 30 g/l ; elle peut être plus particulièrement inférieure à 20 g/l. La concentration en acide chlorhydrique peut être ajustée de telle sorte que le pH de la solution soit stabilisée entre environ 4 et 5.

La cathode utilisée dans le dispositif pour la mise en oeuvre de la section (A) peut être constituée de tiges en acier montées verticalement et soudées sur une plaque circulaire de telle sorte que l'ensemble réalise une géométrie cylindrique. L'anode peut être constituée par un cylindre métallique placé au centre de la cathode, de préférence en tôle déployée. Le cylindre peut être revêtu d'oxyde d'iridium et/ou d'oxyde de ruthénium. L'ensemble cathode et anode peut avoir une hauteur d'environ 300 mm et un diamètre d'environ 300 mm.

Sur la figure 1, la référence 1 désigne la conduite d'alimentation en eau à traiter. Un débitmètre 2 placé sur la conduite mesure en continu le débit d'eau afin d'adapter le traitement au volume d'eau. Le débitmètre est par exemple un compteur à impulsion permettant les mesures de débits jusqu'à 15 m³/h. De préférence, le débitmètre permet la mesure de débit dans la plage de 0,1 à 15 m³/h.
Le dispositif de mise en oeuvre de la section (A) comporte un bac 3 de stockage et de préparation de la solution électrolytique. La solution électrolytique est constituée d'eau distillée, de chlorure de sodium et de l'acide chlorhydrique. La concentration en sel de la solution est comprise entre 20 et 30 g/l. La quantité ajoutée d'acide chlorhydrique est telle qu'elle permet de stabiliser entre environ 4 et 5 le pH de la solution préparée dans le bac 3. De préférence, la solution électrolytique a un pH d'environ 5 et une concentration en chlorure de sodium d'environ 30 g/l.

Le bac de stockage 3 de la solution électrolytique peut être équipé d'une sonde 4 de détection du niveau bas électrolyte, qui déclenche une alarme en cas de manque de produit.
Une pompe d'injection et d'alimentation 5 transfère la solution électrolytique à un débit variable allant en général jusqu'à environ 10 l/h dans le réacteur d'électrolyse (ou électrolyseur) 6. De préférence, ladite pompe est de type « pompe doseuse » et délivre un débit pouvant aller de 500 ml/h à 10 l/h. L'admission de la solution électrolytique se fait de préférence dans le bas de l'électrolyseur. Le débit de la pompe est en général réglé en fonction du débit d'eau dans la canalisation.
Le réacteur d'électrolyse 6 est constitué d'une chambre contenant une anode 7 et une cathode 8 reliées à un générateur 9 de courant à intensité variable pouvant atteindre 140 ampères sous 12 volts. Le réacteur d'électrolyse 6 est équipé d'une double enveloppe 10 dans laquelle circule un fluide de refroidissement, par exemple de l'eau, permettant de maintenir le milieu réactionnel à une température inférieure à 30 °C. De préférence, l'enveloppe du réacteur d'électrolyse est en polypropylène et a une forme cylindrique.

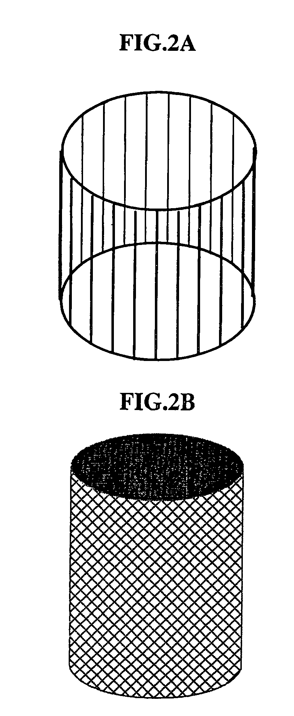

La figure 2A illustre une cathode 8 constituée par un ensemble de tiges 11 en acier inoxydable. Ces tiges sont assemblées verticalement et soudées sur une plaque circulaire en acier inoxydable, de telle sorte que l'ensemble réalise une géométrie cylindrique pour la cathode. L'anode 7 telle qu'illustrée par la figure 2B est en forme de cylindre en titane recouvert d'oxyde mixte d'iridium et de ruthénium est placée au centre de la cage formée par la cathode 8 et du réacteur électrolytique 10. Une telle disposition favorise la réaction électrochimique créée par l'anode et la cathode. Notamment les mouvements des liquides, favorables à la réaction, sont permis grâce aux formes respectives anode/cathode.
Les électrodes utilisées pour le réacteur électrolytique 6 (anode et cathode) peuvent fonctionner avec une densité de courant allant jusqu'à 500 A/m². De préférence, les électrodes sont soumises à des densités de courant variant de 100 à 500 A/m². Les électrodes 7 et 8 sont reliées à un générateur 9 de courant continu stabilisé et filtré délivrant une intensité de courant pouvant être régulée et contrôlée entre 4 et 140 A.

Les paramètres de régulation majeurs dans la section (A) du procédé de l'invention sont la variation du débit d'injection de la solution renfermant le chlore libre en fonction du débit d'alimentation en eau froide et la variation de l'intensité de courant en fonction de la variation du débit d'injection.

La solution ayant subi une transformation électrochimique au sein de l'électrolyseur 6 est injectée dans le réseau d'alimentation en eau froide par l'intermédiaire d'un conduit 12, dont l'orifice est de préférence éloigné de l'alimentation en solution électrolytique et située dans la partie haute de l'électrolyseur 6. Le conduit 12 débouche dans la conduite 1 par une sonde d'injection 13 équipée d'un clapet anti-retour, qui peut être un tube en polypropylène équipé d'un clapet anti-retour sous forme de bille montée sur un ressort.

La solution dite électrolytique introduite dans l'électrolyseur 6, soumise au passage d'un courant continu par des électrodes appropriées devient le siège des réactions suivantes :

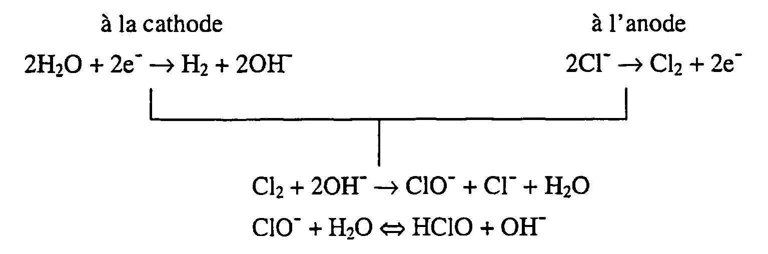

Le chlore libre consiste essentiellement en les espèces HCIO et CIO⁻. L'effet bactéricide est maximal lorsque le chlore est sous forme HCIO, qui est alors appelé chlore libre actif. A des pH alcalins, l'équilibre est favorable à l'espèce ClO⁻, alors qu'à des pH neutres ou acides, l'équilibre est favorable à l'espèce HCIO.

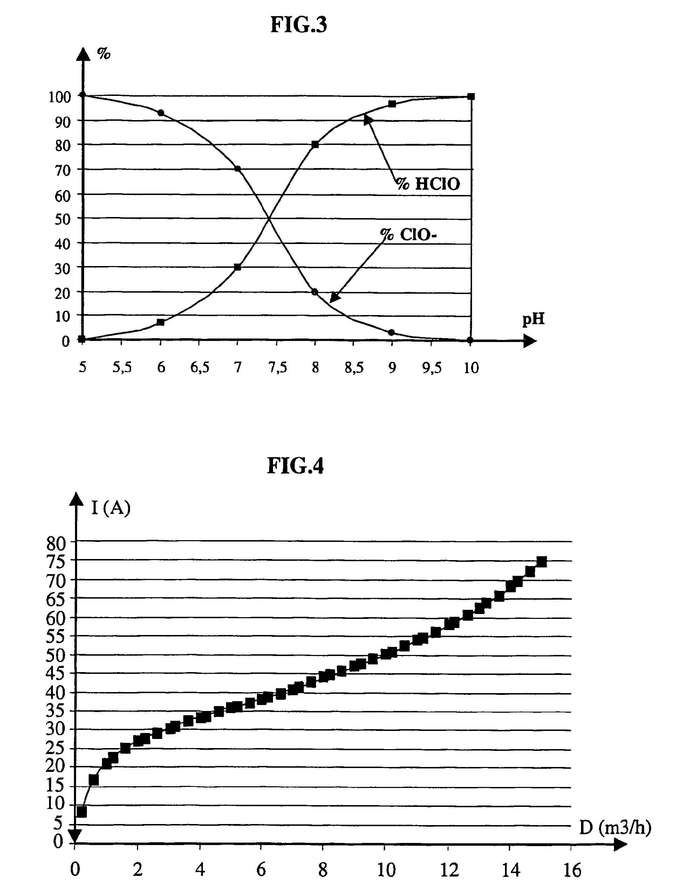

Particulièrement, la mise en oeuvre de la section (A) du procédé de l'invention permet d'obtenir, après électrolyse, par l'ajustage initial du pH initial à environ 5 dans le bac 3 de la solution électrolytique par ajout d'acide chlorhydrique un liquide contenant du chlore libre avec un pH voisin de 7. Ainsi, le chlore libre se trouve à plus de 60 % sous forme de chlore libre actif, contrairement aux procédés classiques aboutissant à des solutions alcalines dont le pH est au mieux égal à 8, valeur pour laquelle le chlore libre n'est qu'à 20 % sous forme de chlore libre actif. La figure 3 donne les courbes de distribution (en pourcentage massique en ordonnées) des espèces HClO et ClO⁻ en fonction du pH, en abscisses. Au pH 7, il y a environ 70 % de HClO.

Conformément à l'invention et grâce à une régulation adaptée de l'intensité de courant et du débit de l'injection en fonction du débit d'alimentation des équipements de stockage et production d'eau chaude, l'augmentation des chlorures dans l'eau n'excède en aucun cas 10 mg/l, suite à l'utilisation d'une solution électrolytique avec une concentration initiale en chlorure ne dépassant pas 30 g/l, demeurant plus particulièrement inférieure à 20 g/l. Ces conditions de fonctionnement protègent des risques de corrosion connus lors de l'injection de chlore libre.

La figure 4 montre un exemple de régulation d'intensité de courant du générateur 9 (I en ampères en ordonnées) en fonction du débit d'alimentation en eau froide (D en m³/h en abscisses).

Le liquide sortant du réacteur électrolytique 6 contient en général de 2 à 4 g/l de chlore libre selon l'intensité de courant et du débit d'injection.
Dans une version préférée de l'invention, on traite le liquide sortant du réacteur électrolytique 6 de manière à lui conférer une concentration en chlore libre allant de 2,5 à 3 g/l.

Selon la présente invention, le débit d'injection du liquide de désinfection et de lutte contre la légionellose est régulé en fonction du débit d'eau froide alimentant les équipements de stockage et de production d'eau chaude, de manière à avoir une concentration de l'eau froide désinfectée en chlore libre allant de 2 à 3,5 mg/l et après son chauffage et sa distribution une concentration en chlore libre allant de 1 à 3 mg/l, sans pour autant dépasser 3 mg/l pour éviter la formation de trihalométhanes.

Avantageusement, le débit d'injection du liquide de désinfection est régulé de telle sorte que l'eau froide renferme de 1,5 à 2,5 mg/l de chlore libre, et de 1 à 2 mg/l de chlore libre après son chauffage et sa distribution, quel que soit le temps de séjour de stockage. Ces gammes de concentration sont fixées conformément à la circulaire de la DGS N°97/311 du 24 avril 1997 relative à la surveillance et à la prévention de la légionellose.

Selon un mode particulier de réalisation du procédé de l'invention, la mise en oeuvre d'une étape (C) permet la protection cathodique contre la corrosion des réservoirs B1 et B2, par courant imposé. Elle permet en outre une génération électrolytique permanente du chlore libre au sein des réservoirs par électrolyse des chlorures résiduels (10 mg/l), selon les réactions déjà décrites, pour compenser l'effet de dismutation du chlore libre favorisé par les températures élevées.
Le dispositif utilisé comprend pour chacun des réservoirs B1 et B2 une cellule électrolytique comprenant une anode, par exemple en tige de titane recouvert d'oxyde mixte d'iridium et de ruthénium montée sur chacun des réservoirs B1 et B2 en assurant une isolation parfaite entre lesdits réservoirs et la dite anode. Chaque cellule électrolytique comprend en outre un générateur de courant à intensité et potentiel contrôlé (redresseur) pouvant délivrer un courant continu, stabilisé et filtré.
Sur la figure 1, les parois des réservoirs B1 et B2 servant de cathodes et les anodes C1 et C2 sont reliées aux générateurs C3 et C4.
Pour chaque réservoir B1 et B2, le dispositif électrolytique de l'étape (C) est le siège d'une densité de courant permettant le maintien de la concentration de l'eau en chlore libre, et la compensation des réactions de dismutation de chlore libre à températures élevées :

2HClO → Cl⁻ + HClO₂ + H⁺

Le dispositif (C) est décrit dans la demande de brevet français FR-A-2 776 370.

Selon l'invention, comme indiqué dans la description qui précède, on peut opérer seulement la section (B) de choc thermique permanent de l'eau produisant une désinfection et une stérilisation de l'eau.
On peut réaliser à titre préventif et curatif seulement l'adjonction continue du chlore libre produit par électrolyse dans l'eau. C'est ce qui est décrit et revendiqué dans la demande de brevet français déposée par les demanderesses sous le numéro FR 00/00 598, le 18 janvier 2000 ( = EP1118586/ FR280384).
Par ailleurs, selon l'invention, le dispositif (B) de choc thermique peut être utilisé pour un choc thermique curatif occasionnel par action sur la vanne mélangeuse V et maintien de la température de l'eau distribuée à 70 °C au minimum dans tout le circuit de distribution conformément à la circulaire de la DGS N° 97/311 du 24 avril 1997.
De même, le mode de production du chlore libre de la section (A) peut être utilisé pour opérer un choc chloré curatif occasionnel de l'eau par modification du débit de la solution électrolytique et maintien de la concentration du chlore libre dans l'eau à une valeur de 12 mg/l selon la circulaire de la DGS N° 97/311 du 24 avril 1997.

L'ensemble des composants du procédé selon l'invention est conçu pour un fonctionnement sous des pressions pouvant atteindre 10 bar.
Le fonctionnement du dispositif objet de l'invention est géré par des moyens électroniques (14 sur la figure 1) comportant une carte électronique permettant le contrôle et la régulation de l'ensemble du système en agissant sur le réglage du courant d'électrolyse du dispositif (A) compte tenu de la valeur du débit d'eau froide mesurée parle débitmètre 2 (la mesure étant symbolisée par la flèche 15). Le débit d'alimentation fourni par la pompe 5, la température de distribution de l'eau, ainsi que le courant injecté dans le dispositif (C) peuvent aussi être contrôlés.
On notera que le présent dispositif de mise en oeuvre du procédé selon l'invention est adapté à la fois à la lutte contre la légionelle et à la distribution d'eau chaude sanitaire sans limitation de débit (hormis bien entendu du fait du dimensionnement initial de l'installation). Aucun dispositif limiteur, ou contrôleur, de débit n'est indispensable pour la bonne marche du procédé.

## Revendications

1. Procédé continu de désinfection et de lutte contre la légionellose dans les équipements de stockage et de production et/ou dans les circuits de distribution d'eau chaude sanitaire **caractérisé en ce qu'**il comprend une section (B) dans laquelle :
- on admet l'eau du réseau dans un réservoir d'admission servant de tampon et après préchauffage de l'eau du réseau par échange thermique avec l'eau contenue dans un réservoir doté d'un moyen de chauffage assurant un choc thermique permanent de l'eau à un niveau de température dans une gamme d'environ 85°C jusqu'à 109°C, au moyen d'un échangeur de chaleur ;
- on admet l'eau préchauffée dans ledit réservoir de choc thermique ;
- on refroidit au moins une partie de l'eau sortant dudit réservoir de choc thermique par échange avec l'eau entrante au travers dudit échangeur de chaleur
- on régule à la température de distribution par mélange de cette eau refroidie avec l'autre partie de l'eau provenant du choc thermique et donc qui n'a pas été refroidie; et
- on la distribue à la température désirée.

2. Procédé selon la revendication 1 **caractérisé en ce que** l'eau du réseau est admise dans ledit réservoir d'admission sous une pression allant jusqu'à 10 bar.

3. Procédé selon l'une des revendications 1 ou 2 **caractérisé en ce que** l'eau préchauffée séjourne dans ledit réservoir de choc thermique pendant au moins 30 minutes.

4. Procédé selon l'une des revendications 1 à 3 **caractérisé en ce que** l'on opère un choc thermique curatif occasionnel par régulation de la température de distribution de l'eau chaude par admission dans l'eau à distribuer d'une proportion d'eau très chaude du réservoir de choc thermique.

5. Procédé selon l'une des revendications 1 à 4 **caractérisé en ce qu'**il comprend en outre une section (A) dans laquelle on produit en continu une solution électrolytique contenant du chlore libre et on l'introduit dans l'eau à traiter par choc thermique.

6. Procédé selon la revendication 5 **caractérisé en ce que** l'on obtient une eau chaude à la sortie des robinets avec une concentration en chlore libre n'excédant pas 3mg/l par l'automatisation et le contrôle électronique des paramètres de fonctionnement.

7. Procédé selon l'une des revendications 5 et 6 **caractérisé en ce que** l'on s'assure que le taux d'augmentation des chlorures dans l'eau n'excède pas 10 mg/l par l'automatisation et le contrôle électronique des paramètres de fonctionnement.

8. Procédé selon l'une des revendications 5 à 7 **caractérisé en ce que** l'on opère un choc chloré curatif occasionnel de l'eau par modification du débit de la solution électrolytique et maintien de la concentration en chlore libre dans l'eau à une valeur de 12 mg/l selon la circulaire de la DGS N°97/311 du 24 avril 1997.

9. Procédé selon l'une des revendications 1 à 8 **caractérisé en ce qu'**il comporte en outre une section (C) dans laquelle on réalise la protection cathodique des réservoirs contre la corrosion, par courant imposé, et la génération permanente de chlore libre à partir des chlorures résiduels.

10. Dispositif pour la mise en oeuvre d'un procédé selon l'une des revendications 1 à 9 **caractérisé en ce qu'**il comporte, dans la section (B),
- au moins un réservoir d'admission de l'eau B1,
- un réservoir de choc thermique B2 dimensionné pour la réalisation d'un choc thermique de l'eau à un niveau de température dans une gamme d'environ 85°C jusqu'à 109°C pendant au moins 30 minutes et équipé d'un moyen de production du choc énergétique du choc thermique et de chauffage B4 pour le maintien de la température de l'eau à une valeur comprise entre 85 et 109°C,
- un moyen d'échange de chaleur par (B3) entre l'eau de réseau et une proportion de l'eau provenant du choc thermique (B21), des moyens d'introduction de cette eau préchauffée dans (B1) étant prévus,
- une vanne de régulation et de mélange V entre l'eau refroidie (B22) et une autre proportion de l'eau provenant du choc thermique, la vanne V est contrôlée par des moyens de contrôle T de la température de distribution d'eau
- des moyens de mesure de température du choc thermique dans le réservoir B2.

11. Dispositif selon la revendication 10 dans lequel ledit réservoir B1 est adapté pour le stockage de l'eau à traiter.

12. Dispositif selon l'une des revendications 10 ou 11 dans lequel l'échangeur B3est adapté à effectuer le refroidissement de l'eau ayant subi ledit choc thermique par l'eau froide entrant dans le procédé.

13. Dispositif selon l'une des revendications 10 à 12 dans lequel ladite vanne de mélange est une vanne trois voies permettant l'admission de l'eau très chaude en provenance du réservoir B2 pour réguler la température de distribution d'eau chaude.

14. Dispositif selon l'une des revendications 10 à 13, pour la mise en oeuvre d'un procédé selon la revendication 9, **caractérisé en ce qu'**il comporte, dans la section (C), au moins une anode C1 et C2 pour chaque réservoir B1 et B2, un générateur de courant C3 ou C4, un moyen de contrôle du courant électrique injecté par le générateur C3 ou C4.

## Claims

1. A continuous disinfection process for combatting legionellosis in storage and production equipment and/or in circuits for distributing hot domestic supply water **characterised in that** it includes a section (B) in which:
- system water is admitted into an intake tank, which acts as a buffer, and after the system water is preheated by heat exchange with water contained in a tank provided with a heating means which provides a permanent water heat shock at a temperature level in a range of approximately 85°C to 109°C, by means of a heat exchanger;
- the preheated water is admitted into said heat shock tank;
- at least part of the water leaving said heat shock tank is cooled by exchange with the water entering through said heat exchanger;
- regulation to the distribution temperature takes place by mixing this cooled water with the other part of the water which results from the heat shock and thus which has not been cooled; and
- it is distributed at the desired temperature.

2. The process according to Claim 1, **characterised in that** the system water is admitted into said intake tank at a pressure which reaches 10 bar.

3. The process according to one of claims 1 or 2, **characterised in that** the preheated water stays in said heat shock tank for at least 30 minutes.

4. The process according to one of claims 1 to 3, **characterised in that** an occasional curative heat shock is effected by regulating the distribution temperature of the hot water by admitting into the water to be distributed a proportion of very hot water from the heat shock tank.

5. The process according to one of claims 1 to 4, **characterised in that** it further includes a section (A) in which an electrolytic solution containing free chlorine is continuously produced and is introduced into the water to be treated by heat shock.

6. The process according to Claim 5, **characterised in that** hot water is obtained at the tap outlet with a free chlorine concentration which does not exceed 3mg/l by means of the automation and electronic control of the functioning parameters.

7. The process according to one of the claims 5 and 6, **characterised in that** it is ensured that the rate of increase of the chlorides in the water does not exceed 10mg/l by means of the automation and electronic control of the functioning parameters.

8. The process according to one of claims 5 to 7, **characterised in that** an occasional curative chlorine shock of water is controlled by modifying the flow of the electrolytic solution and keeping the concentration of free chlorine in water at a value of 12mg/l in accordance with the community health protection (DGS) circular No. 97/311 of 24th April 1997.

9. The process according to one of claims 1 to 8, **characterised in that** it further comprises a section (C) in which cathodic protection of the tanks against corrosion, by means of impressed current, and the permanent generation of free chlorine from residual chlorides are brought about.

10. A device for implementing a process according to one of claims 1 to 9, **characterised in that** it comprises, in section (B),
- at least one water intake tank B1,
- one heat shock tank B2 which is sized to bring about a water heat shock at a temperature level in a range of approximately 85°C to 109°C over at least 30 minutes and equipped with a means for producing energy shock for the heat shock and a heating means B4 for maintaining the temperature of the water at a value of between 85 and 109°C,
- a means for exchanging heat by means of (B3) between the system water and a proportion of the water resulting from heat shock (B21), the means for introducing this water which is preheated in (B1) being provided,
- a control and mixing valve V between the cooled water (B22) and another proportion of the water resulting from the heat shock, the valve V is controlled by the means T for controlling the temperature of the water distribution
- means for measuring the temperature of the heat shock in tank B2.

11. The device according to Claim 10 in which said tank B1 is adapted to store the water which is to be treated.

12. The device according to one of claims 10 or 11 in which the exchanger B3 is adapted to bring about the cooling of the water which has been subjected to said heat shock by cold water entering into the process.

13. The device according to one of claims 10 to 12 in which said mixing valve is a three-way valve which makes possible the ingress of very hot water from the tank B2 in order to regulate the hot water distribution temperature.

14. The device according to one of claims 10 to 13, for the implementation of a process according to Claim 9,
**characterised in that** it comprises, in section (C), at least one anode C1 and C2 for each tank B1 and B2, a current generator C3 or C4, a means for controlling the electric current provided by the generator C3 or C4.

## Patentansprüche

1. Kontinuierliches Desinfektionsverfahren gegen Legionellose in Einrichtungen zur Warmwasserspeicherung und -bereitung und/oder in Warmwasserverteilungskreisen, **dadurch gekennzeichnet, dass** es einen Abschnitt (B) umfasst, in dem:
- das Wasser aus dem Leitungsnetz in einem Aufnahmebehälter aufgenommen wird, der als Zwischenspeicher dient, und nach einem Vorwärmen des Wassers aus dem Leitungsnetz durch Wärmeaustausch mit dem Wasser, das in einem Behälter enthalten ist, der mit Heizmitteln versehen ist, die einen permanenten Wärmeschock des Wassers bei einer Temperatur in einem Bereich von ungefähr 85 °C bis 109°C sicherstellen, mittels eines Wärmetauschers
- das vorgewärmte Wasser in dem Wärmeschockbehälter aufgenommen wird;
- mindestens ein Teil des Wassers, das den Wärmeschockbehälter verlässt, durch Wärmeaustausch mit dem hereinkommenden Wasser im Wärmetauscher abgekühlt wird;
- die Verteilungstemperatur durch Mischen dieses abgekühlten Wassers mit dem anderen Teil des Wassers, der aus dem Wärmeschockbehälter kommt und folglich nicht abgekühlt worden ist, reguliert wird; und
- das Wasser mit der gewünschten Temperatur verteilt wird.

2. Verfahren nach Anspruch 1, **dadurch gekennzeichnet, dass** das Wasser aus dem Leitungsnetz in dem Aufnahmebehälter unter einem Druck, der bis zu 10 bar erreichen kann, aufgenommen wird.

3. Verfahren nach einem der Ansprüche 1 oder 2, **dadurch gekennzeichnet, dass** das vorgewärmte Wasser mindestens 30 Minuten lang in dem Wärmeschockbehälter bleibt.

4. Verfahren nach einem der Ansprüche 1 bis 3, **dadurch gekennzeichnet, dass** ein gelegentlicher Krankheitskeime abtötender Wärmeschock durch Regulieren der Verteilungstemperatur des warmen Wassers durch Aufnehmen einen Anteils sehr heißen Wassers aus dem Wärmeschockbehälter in das zu verteilende Wasser bewirkt wird.

5. Verfahren nach einem der Ansprüche 1 bis 4, **dadurch gekennzeichnet, dass** es außerdem einen Abschnitt (A) umfasst, in dem kontinuierlich eine Elektrolytlösung erzeugt wird, die ungebundenes Chlor enthält, und in das mittels Wärmeschock zu behandelnde Wasser eingeleitet wird.

6. Verfahren nach Anspruch 5, **dadurch gekennzeichnet, dass**, durch Automatisierung und elektronische Steuerung der Betriebsparameter, am Auslass der Wasserhähne ein Warmwasser mit einer Konzentration an ungebundenem Chlor erhalten wird, die 3 mg/l nicht übersteigt.

7. Verfahren nach einem der Ansprüche 5 und 6, **dadurch gekennzeichnet, dass** durch Automatisierung und elektronische Steuerung der Betriebsparameter sichergestellt wird, dass die Zuwachsrate bei den Chloriden im Wasser 10 mg/l nicht übersteigt.

8. Verfahren nach einem der Ansprüche 5 bis 7, **dadurch gekennzeichnet, dass** ein gelegentlicher Krankheitskeime abtötender Chlor-Schock des Wasser durch Modifizieren des Volumenstroms der Elektrolytlösung und Aufrechterhalten der Konzentration an ungebundenem Chlor im Wasser auf einem Wert von 12 mg/l, gemäß DGS-Rundschreiben Nr. 97/311 vom 24. April 1997, bewirkt wird.

9. Verfahren nach einem der Ansprüche 1 bis 8, **dadurch gekennzeichnet, dass** es außerdem einen Abschnitt (C) aufweist, in dem ein kathodischer Korrosionsschutz der Behälter durch einen aufgezwungenen Strom und die ständige Erzeugung von ungebundenem Chlor aus den Rest-Chloriden verwirklicht wird.

10. Vorrichtung zum Durchführen eines Verfahrens nach einem der Ansprüche 1 bis 9, **dadurch gekennzeichnet**, das sie im Abschnitt (B) umfasst:
- mindestens einen Wasseraufnahmebehälter B1,
- einen Wärmeschockbehälter B2, der für die Verwirklichung eines Wärmeschocks des Wassers bei einer Temperatur im Bereich von ungefähr 85 °C bis 109 °C während mindestens 30 Minuten ausgelegt ist und mit Mitteln B4 zum Hervorrufen des energetischen Schocks für den Wärmeschock und die Erwärmung, um die Temperatur des Wassers auf einem Wert im Bereich zwischen 85 und 109 °C zu halten, ausgestattet ist,
- Mittel (B3) zum Wärmeaustausch zwischen dem Wasser aus dem Leitungsnetz und einem Teil des Wassers, das aus dem Wärmeschockbehälter kommt (B21), wobei Mittel zum Einleiten dieses vorgewärmten Wassers in (B1) vorgesehen sind,
- ein Regulier- und Mischventil V zwischen dem abgekühlten Wasser (B22) und einem anderen Teil des Wassers, das aus dem Wärmeschockbehälter kommt; wobei das Ventil V durch Mittel T zum Steuern der Wasserverteilungstemperatur gesteuert wird,
- Mittel zum Messen der Temperatur des Wärmeschocks im Behälter B2.

11. Vorrichtung nach Anspruch 10, bei der der Behälter B1 für die Speicherung des zu behandelnden Wassers ausgelegt ist.

12. Vorrichtung nach einem der Ansprüche 10 oder 11, bei der der Wärmetauscher B3 dafür ausgelegt ist, ein Abkühlen des Wassers, das den Wärmeschock erfahren hat, mit kaltem Wasser, das in das Verfahren eintritt, durchzuführen.

13. Vorrichtung nach einem der Ansprüche 10 bis 12, bei der das Mischventil ein Dreiwegeventil ist, das das Einströmen von sehr heißem Wasser aus dem Behälter B2 ermöglicht, um die Warmwasserverteilungstemperatur zu regulieren.

14. Vorrichtung nach einem der Ansprüche 10 bis 13 für die Durchführung eines Verfahrens nach Anspruch 9, **dadurch gekennzeichnet, dass** sie im Abschnitt (C) mindestens eine Anode C1 bzw. C2 für jeden Behälter B1 und B2, einen Stromgenerator C3 oder C4, Mittel zum Steuern des elektrischen Stroms, der durch den Generator C2 oder C4 eingespeist wird, umfasst.
